# EUROPEAN PATENT APPLICATION

(11) **EP 4 779 297 A1**
(43) Date of publication of application: **22.07.2026**
(21) Application number: 25227272.9
(22) Date of filing: 25.12.2025
(51) Int. Cl.: G01N 21/85, G01N 21/25, G01N 21/27, G01N 21/31, A61M 5/168, G01N 21/359

(54) **SPECTROSCOPIC LIQUID FLOW ANALYSIS DEVICE FOR MEASURING SUBSTANCE CONCENTRATIONS**

(30) Priority: 10.01.2025 US 202563743908 P; 08.12.2025 US 202519412031
(71) Applicant: Honeywell International Inc., Charlotte, NC 28202 (US)
(72) Inventor: YEE, Daniel James, Charlotte, 28202 (US); BROWN, Andy Walker, Charlotte, 28202 (US); WADE, Richard A, Charlotte, 28202 (US); SHAFAI, Moin S, Charlotte, 28202 (US)
(74) Representative: Haseltine Lake Kempner LLP

(57) **Abstract**

A spectroscopic liquid flow device coupled to a liquid flow path, wherein the spectroscopic liquid flow device comprises a measurement cell configured to receive at least a portion of the liquid flow path; a laser emitter configured to (i) provide a dual frequency comb source, (ii) generate a spectrum of optical frequencies, and (iii) emit the spectrum of optical frequencies through the portion of the liquid flow path in the measurement cell; and a photodetector configured to (i) receive the spectrum of optical frequencies passed through the portion of the liquid flow path and (ii) generate an output signal based on the spectrum of optical frequencies passed through the portion of the liquid flow path; and a computing system configured to determine one or more substance concentrations in the liquid flow path based on the output signal.

## Description

### CROSS REFERENCE TO RELATED APPLICATION

This application claims the priority of U.S. Provisional Application No. 63/743,908, entitled "SPECTROSCOPIC LIQUID FLOW ANALYSIS DEVICE FOR MEASURING SUBSTANCE CONCENTRATIONS," filed on January 10, 2025, the disclosure of which is hereby incorporated by reference in its entirety.

### BACKGROUND

Variability in drug dosages can lead to adverse effects, ineffective treatment, increased healthcare costs, and in severe cases, patient mortality. Current methods lack the precision necessary for safe dosing, resulting in potential dosing errors. The complexity of drug delivery systems, including multiple infusion pumps and variations in tube set lengths, exacerbates the risk of complications and errors. Applicant has identified many technical challenges and difficulties associated with conventional drug delivery systems.

### BRIEF SUMMARY

Various embodiments described herein relate to components, apparatuses, and systems for monitoring drug concentration in intravenous and infusion therapies. According to some embodiments, a system comprises a spectroscopic liquid flow device coupled to a liquid flow path, wherein the spectroscopic liquid flow device comprises a measurement cell configured to receive at least a portion of the liquid flow path; a laser emitter configured to (i) provide a dual frequency comb source, (ii) generate a spectrum of optical frequencies, and (iii) emit the spectrum of optical frequencies through the portion of the liquid flow path in the measurement cell; and a photodetector configured to (i) measure one or more absorption and wavelength characteristics of the spectrum of optical frequencies passed through the portion of the liquid flow path and (ii) generate an output signal based on the one or more absorption and wavelength characteristics; and a computing system configured to determine one or more substance concentrations in the liquid flow path based on the output signal.

In some embodiments, the measurement cell comprises a channel with a thickness of approximately 1 mm and an optic window with a width of approximately 4 mm. In some embodiments, the dual frequency comb source comprises two optical frequency combs that are configured to emit laser light at evenly spaced intervals across the spectrum of optical frequencies. In some embodiments, the liquid flow path is configured to transport a substance from a drug delivery source, wherein the drug delivery source comprises at least one of an infusion pump, a syringe pump, an intravenous bag, or a dialysis machine. In some embodiments, the photodetector is configured to measure absorption and wavelength characteristics of the spectrum of optical frequencies passed through the portion of the liquid flow path. In some embodiments, the output signal corresponds to at least one of dosage, concentration, composition, volume, or flow rate of the liquid flow path. In some embodiments, (i) the computing system comprises a machine learning algorithm that is configured to determine at least one of active pharmaceutical ingredients or excipients in the liquid flow path based on the output signal and (ii) the computing system is configured to determine the one or more substance concentrations by comparing the output signal with one or more reference spectra.

According to some embodiments, a spectroscopic liquid flow device comprises: an optical coupling system comprising a laser emitter and a measurement cell, wherein (i) the laser emitter provides a dual frequency comb source, (ii) the laser emitter is configured to emit a laser light comprising a spectrum of optical frequencies, (iii) the optical coupling system is configured to direct the laser light from the laser emitter through the measurement cell, and (iv) the measurement cell is configured to receive a liquid flow; a detection system configured to (i) capture transmitted light from the measurement cell and (ii) generate a data signal representative of one or more measured light spectra from the transmitted light; a spectral processing unit configured to generate an output signal based on the data signal.

In some embodiments, the optical coupling system comprises one or more lenses or one or more optical fibers that direct the laser light into the liquid flow within the measurement cell. In some embodiments, the measurement cell is configured to hold at least a portion of the liquid flow comprising a liquid sample while allowing the laser light to pass through the liquid sample. In some embodiments, the detection system comprises at least one of a photodetector or a camera system. In some embodiments, the spectral processing unit is configured to interpret the output signal and quantify one or more concentrations of one or more components within the liquid flow. In some embodiments, the spectroscopic liquid flow device further comprises a calibration and reference system that is configured to control or vary one or more substance concentrations in the liquid flow based on the output signal. In some embodiments, the calibration and reference system (i) comprises one or more samples or one or more reference spectra, and (ii) is configured to calibrate the output signal based on the one or more samples or the one or more reference spectra.

According to some embodiments, a computer-implemented method comprises generating, by one or more processors, a spectrum of optical frequencies with a laser light from a dual frequency comb source; generating, by the one or more processors, an output signal based on the spectrum of optical frequencies passing through a measurement cell comprising a liquid flow; and determining, by the one or more processors, one or more substance concentrations in the liquid flow based on the output signal.

In some embodiments, generating the spectrum of optical frequencies comprises providing the laser light at evenly spaced intervals across a spectrum. In some embodiments, generating the output signal comprises measuring the spectrum of optical frequencies using a detection system that is configured to capture intensity of the spectrum of optical frequencies at a plurality of frequencies. In some embodiments, determining the one or more substance concentrations comprises comparing one or more measured light spectra corresponding to the output signal to one or more reference spectra. In some embodiments, (i) determining the one or more substance concentrations comprises analyzing, using one or more signal processing algorithms, the output signal, and (ii) the one or more signal processing algorithms comprise a Fourier transform or spectral calibration that identify or quantify absorption features associated with target analytes. In some embodiments, the one or more substance concentrations comprise concentrations of one or more drugs, medications, active pharmaceutical ingredients, or excipients.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 depicts an example drug delivery system;
FIG. 2 depicts example components of a spectroscopic liquid flow analysis system in accordance with some embodiments of the present disclosure;
FIG. 3 depicts an example spectroscopic liquid flow analysis system in accordance with some embodiments of the present disclosure;
FIG. 4 depicts example components of a spectroscopic liquid flow device in accordance with some embodiments of the present disclosure; and
FIG. 5 depicts a flow diagram of an example process for measuring concentrations in a liquid flow in accordance with some example embodiments of the present disclosure.

### DETAILED DESCRIPTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," etc., are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

As described above, there are many technical challenges and difficulties associated with conventional drug delivery systems. With the rise of personalized medicine, accurate and real-time measurement of medication concentration has become critical. Enhancing drug delivery may improve patient safety, treatment efficacy, as well as reduce overall healthcare expenses. Accordingly, a demand exists for improved drug delivery monitoring solutions to provide improved accuracy of drug delivery in clinical settings.

Various embodiments of the present disclosure overcome technical challenges and difficulties in conventional drug delivery systems and provide various technical advancements and improvements. To do so, spectroscopic liquid flow analysis techniques may be used to analyze medications administered via infusion or intravenous methods. By examining molecular composition of drugs and correlating the molecular composition with flow data, an accurate assessment of a total drug dosage is performed before a drug enters a patient's bloodstream. Accordingly, some embodiments of the present disclosure enhance the reliability of drug delivery, thereby improving patient outcomes while reducing the risk of adverse effects and lowering healthcare costs.

More particularly, various embodiments of the present disclosure may address a need for accurate drug concentration monitoring in intravenous and infusion therapies. In some embodiments, spectroscopy is used for measuring medication concentration during drug delivery. Spectroscopy may enable high-resolution, real-time monitoring of drug levels in infusion and intravenous systems by utilizing simultaneous wavelength measurements to capture multiple spectral features, thereby enabling accurate detection of various drug types including small molecules and biologics. As such, measurement precision and sensitivity for drug concentrations may be enhanced, allowing for real-time adjustments to dosing. Spectroscopy may also allow for detection of minute concentration changes, facilitating personalized medicine approaches and improving patient outcomes by ensuring optimal therapeutic levels during treatment along with minimizing patient discomfort via a non-invasive approach.

Various embodiments of the present disclosure may further provide cost and/or resource savings by reducing the need for multiple monitoring devices. That is, usage of spectroscopy may facilitate integration of various measurements into one system or device, thereby lowering equipment costs and maintenance. Components of example components for improving drug delivery system performance are disclosed herewith.

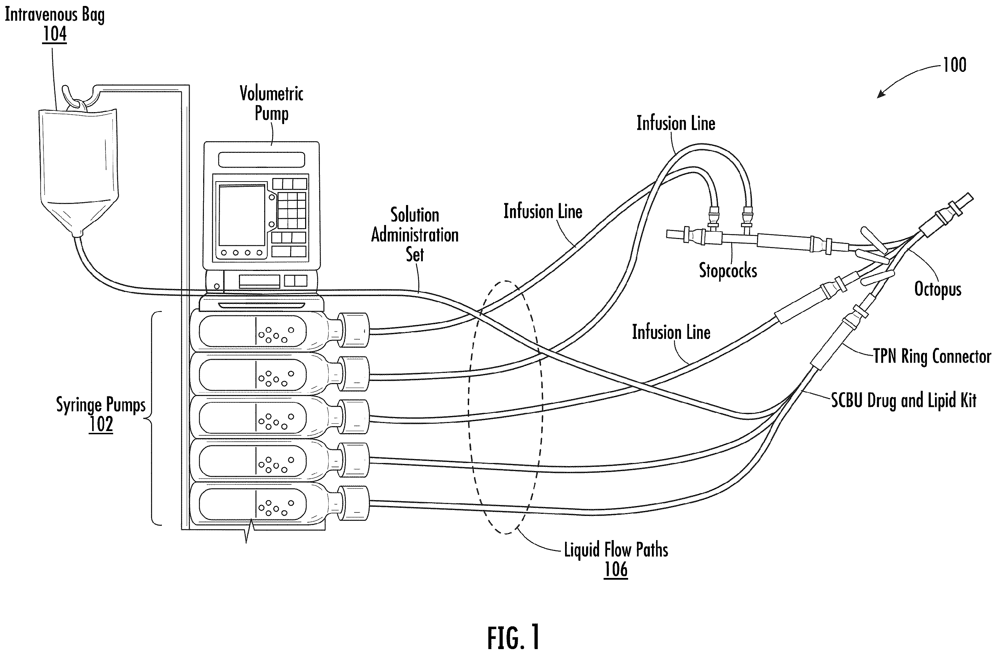

Referring now to FIG. 1, an example drug delivery system 100 is depicted. The drug delivery system 100 may provide a plurality of liquid flow paths 106 comprising substances (e.g., chemical elements and/or compounds) from a plurality of drug delivery sources (e.g., syringe pumps 102 and/or intravenous bag 104). Accordingly, the plurality of liquid flow paths 106 may be analyzed via spectroscopic liquid flow analysis techniques to determine substance concentrations in the plurality of liquid flow paths 106.

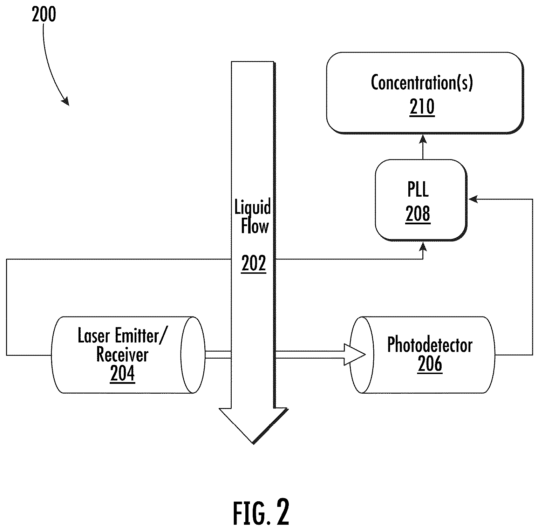

FIG. 2 depicts example components of a spectroscopic liquid flow analysis system 200 in accordance with some embodiments of the present disclosure. The spectroscopic liquid flow analysis system 200 comprises a liquid flow 202 that is configured between a laser emitter/receiver 204 and a photodetector 206. The laser emitter/receiver 204 and the photodetector 206 are configured to provide output signals that may be used by a phase-locked loop (PLL) control system 208 to generate output signals. The PLL control system 208 may use the output signals to control and/or vary one or more concentrations 210 of substances provided in the liquid flow 202. For example, PLL control system 208 may provide a drug delivery system corresponding to the one or more concentrations 210 with output signals comprising feedback to precisely control the timing and/or rate of drug release based on real-time monitoring of the liquid flow 202, thereby "locking" drug delivery to the phase of a signal associated with the liquid flow 202, detected using the laser emitter/receiver 204 and the photodetector 206, and/or the one or more concentrations 210, allowing for highly targeted and responsive medication administration.

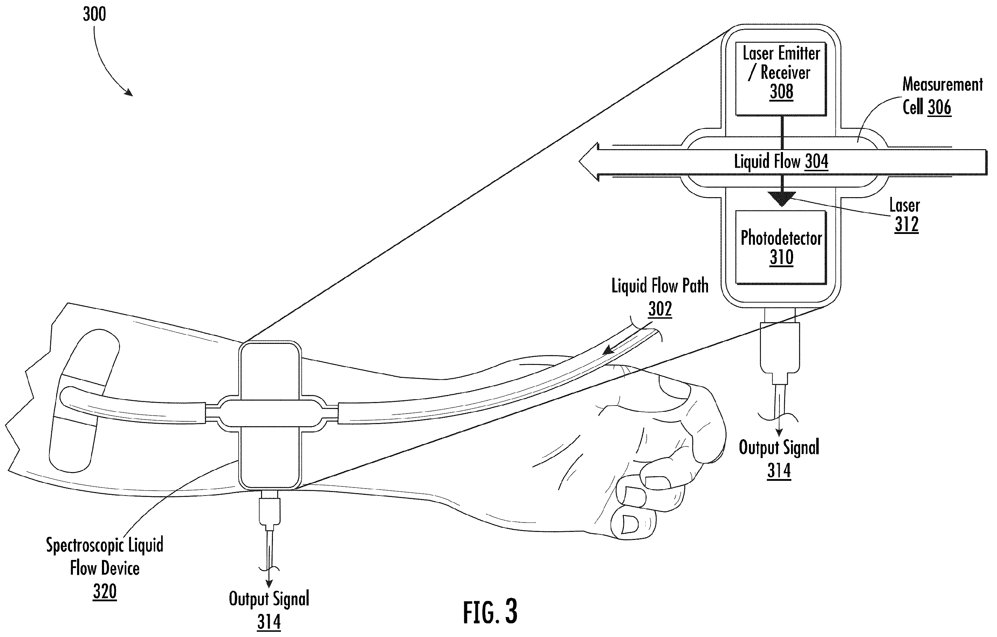

FIG. 3 depicts an example spectroscopic liquid flow analysis system 300 in accordance with some embodiments of the present disclosure. A liquid flow path 302 connects from an infusion pump, syringe, IV bag, dialysis machine, or any drug delivery system (such as drug delivery system 100) to a spectroscopic liquid flow device 320 comprising a measurement cell 306. For example, the measurement cell 306 may comprise a disposable medium comprising a ±1mm thick channel and a 4mm optic window. The spectroscopic liquid flow device 320 further comprises a laser-emitter/receiver 308 and a photodetector 310. The measurement cell 306 is integrated into an optic path between the laser-emitter/receiver 308 (e.g., using dual frequency comb spectroscopy) and the photodetector 310. The laser-emitter/receiver 308 emits a laser light 312 (e.g., comprising a spectrum of optical frequencies) through the measurement cell 306 (e.g., comprising a 4mm optic window) towards the photodetector 310.

The photodetector 310 may be configured to facilitate absorption spectroscopy. For example,, the photodetector 310 may be configured to measure one or more absorption and wavelength characteristics of the laser light 312 as it passes through a composition of the liquid flow 304. That is, passing the laser light 312 through the liquid flow 304 may cause changes in the properties of the laser light 312 that is detected by the photodetector 310 to generate an output signal 314. As such, the output signal 314 may correspond to one or more properties of the liquid flow 304 itself, such as dosage, concentration, and/or composition, as well as volume and/or flow rate. In some embodiments, a camera and/or photodetector of the laser-emitter/receiver 308 is additionally, and/or alternatively (e.g., separate from the photodetector 310) configured to measure scattering/turbidity and/or bubbles of the liquid flow 304. In some embodiments, the camera and/or photodetector of the laser-emitter/receiver 308 may directly measure at least a portion of the laser light 312 that does not pass through the liquid flow 304 to assist with signal processing (e.g., for signal normalization).

In some embodiments, the output signal 314 may be provided by the spectroscopic liquid flow device 320 to a computing system comprising a machine learning algorithm that identifies and/or measures the concentration of multiple drugs (e.g., substance concentrations) in the liquid flow path 302. For example, active pharmaceutical ingredients and/or excipients, and their corresponding amounts, may be identified based on the output signal 314. Accordingly, in situ analysis may be performed on the liquid flow path 302 prior to administration of drug dosage.

In some embodiments, artificial intelligence and/or machine learning algorithms are used to analyze the liquid flow path 302 by comparing the output signal 314 (e.g., generated by the photodetector 310) with the laser light 312 emitted from the laser-emitter/receiver 308. For example, machine learning algorithms, such as linear regression, logistic regression, decision trees, support vector machine (SVM), Naive Bayes, k-nearest neighbors (KNN), k-means, random forest, recurrent neural network (RNN), generative adversarial network (GAN), artificial neural network, and/or the like, may be used to train a predictive model that generates predictions of one or more properties of the liquid flow 304 based on the output signal 314 and one or more properties of the laser light 312 (e.g., molecular absorption, dispersion, speed, frequency, wavelength, intensity, amplitude response, and/or phase response).

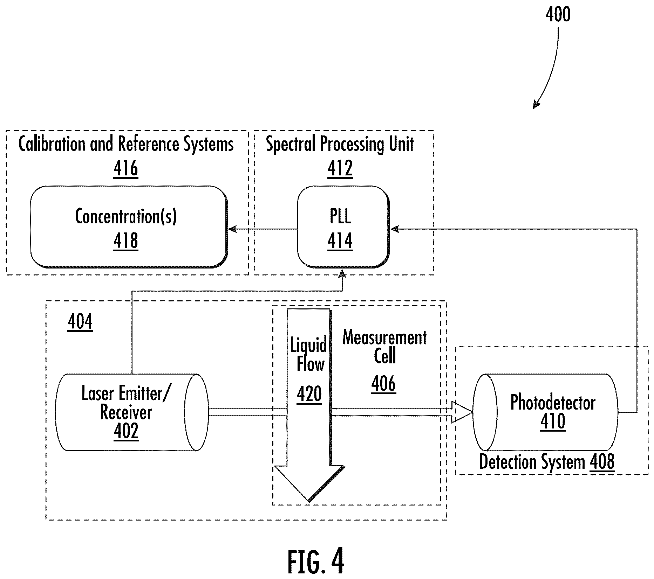

FIG. 4 depicts example components of a spectroscopic liquid flow device 400 in accordance with some embodiments of the present disclosure. The spectroscopic liquid flow device 400 is an example of the spectroscopic liquid flow device 320 in FIG. 3. The spectroscopic liquid flow device 400 comprises a laser emitter/receive 402 (e.g., laser emitter/receiver 204) that provides a dual frequency comb source. In some embodiments, the dual frequency comb source comprises two optical frequency combs that emit laser light at precise, evenly spaced intervals across a broad spectrum, providing high-resolution spectral coverage for analyzing multiple compounds simultaneously.

The laser emitter/receive 402 is configured with a measurement cell 406 (e.g., the measurement cell 306) within an optical coupling system 404. The optical coupling system 404 may comprise components, such as lenses and/or optical fibers that are used to direct and couple emitted laser light from laser emitter/receive 402 into a liquid sample provided by a liquid flow 420 (e.g., liquid flow 202) within the measurement cell 406 for ensuring efficient interaction between the emitted light and analytes. The measurement cell 406 is configured to hold the liquid sample while allowing laser light from the laser emitter/receive 402 to pass through the liquid sample, ensuring minimal optical losses and controlling interaction length.

The spectroscopic liquid flow device 400 further comprises a detection system 408. The detection system 408 comprises a photodetector 410 (e.g., photodetector 206) and/or camera system that is configured to capture transmitted laser light from the emitted laser light passed through the liquid sample in the measurement cell 406, for example, for recording light intensities at a plurality of frequencies. The photodetector 410 may provide data signals representative of measured light spectra from the transmitted laser light captured from the measurement cell 406 to a spectral processing unit 412.

The spectral processing unit 412 may comprise a computer or processing device with software for signal processing and data analysis. The spectral processing unit 412 may utilize algorithms to interpret data signals from the photodetector 410 and quantify concentrations of analyzed/determined components within the liquid sample. The spectral processing unit 412 comprises a PLL control system 414 (e.g., PLL control system 208) that generates output signals based on an analysis of the data signals provided from the photodetector 410. The output signals may be provided to one or more calibration and reference systems 416 to control and/or vary one or more concentrations 418 of substances provided in the liquid flow 420. In some embodiments, the one or more calibration and reference systems 416 comprise samples and/or reference spectra that are used by the one or more calibration and reference systems 416 to calibrate the output signals from PLL control system 414 to provide improved accuracy of identification and/or quantification of analytes in the liquid sample.

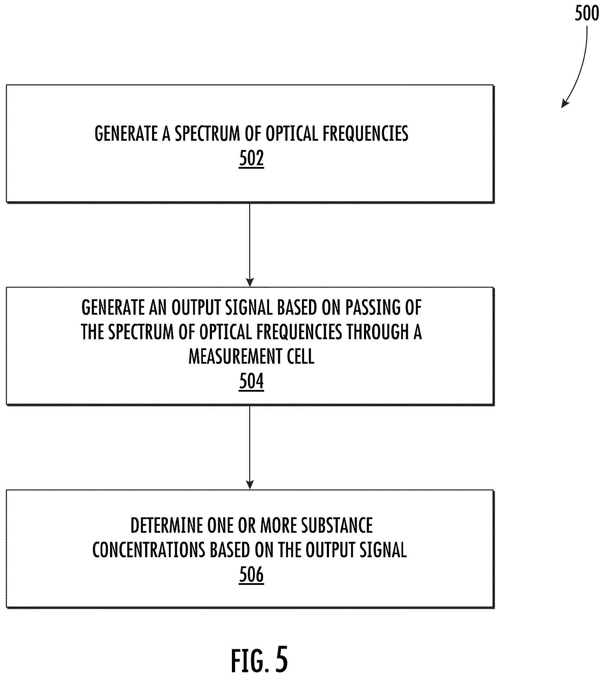

FIG. 5 depicts a flow diagram of an example process 500 for measuring concentrations in a liquid flow in accordance with some example embodiments of the present disclosure. It is noted that each block of a flowchart, and combinations of blocks in the flowchart, may be implemented by various means such as hardware, firmware, circuitry, and/or other devices associated with execution of software including one or more computer program instructions. For example, one or more of the steps/operations described in FIG. 5 may be embodied by computer program instructions, which may be stored by a non-transitory memory of an apparatus employing an embodiment of the present disclosure and executed by a processor component in an apparatus. For example, these computer program instructions may direct the processor component to function in a particular manner, such that the instructions stored in the computer-readable storage memory produce an article of manufacture, the execution of which implements the function specified in the flowchart block(s).

Via the steps/operations of process 500, molecular analysis is performed on a liquid flow with respect to concentration of total drug delivery to a patient by an infusion and/or drug delivery system. In some embodiments, the molecular analysis comprises using a spectroscopy technique that enhances measurement precision and sensitivity for drug concentrations, allowing for real-time adjustments to dosing. In some embodiments, the spectroscopy technique is used to provide information on molecular composition of drugs/medications that are administered to a patient by an infusion and/or drug delivery system.

In some embodiments, the process 500 begins at step/operation 502, where a spectrum of optical frequencies is generated. The spectrum of optical frequencies may be provided by a dual frequency comb source comprising two optical frequency combs that emit laser light at precise, evenly spaced intervals across a broad spectrum. For example, the spectrum of optical frequencies may be applied to a liquid sample comprising analyte flowing through a measurement cell (e.g., a measurement cell 406) by causing a pattern of evenly spaced spectral lines to interact with molecules of the liquid sample in the measurement cell, which may expose unique absorption and/or scattering signatures of the liquid sample at specific wavelengths. In some embodiments, generating the spectrum of optical frequencies comprises simultaneous multi-color imaging at sub-100 nm resolution and at video-rates.

In some embodiments, subsequent to step/operation 502, the example process proceeds to step/operation 504, where an output signal is generated based on passing a laser light comprising the spectrum of optical frequencies through a measurement cell. In some embodiments, a detection system generates the output signal by measuring the spectrum of optical frequencies. In some embodiments, the detection system comprises a photodetector array that is configured to capture and/or measure, from the measurement cell, transmitted light comprising the passed spectrum of optical frequencies at a plurality of frequencies. The detection system may comprise signal processing algorithms that are used to analyze the passed spectrum of optical frequencies by employing techniques, such as Fourier transforms and spectral calibration to identify and quantify absorption features that are associated with target analytes. In some embodiments, the output signal comprises a measured spectra of the spectrum of optical frequencies passed through the measurement cell.

In some embodiments, subsequent to step/operation 504, the example process proceeds to step/operation 506, where one or more substance concentrations are determined based on the output signal. For example, a measured spectra from the output signal may be compared to reference spectra corresponding to substances (e.g., drugs, medications, active pharmaceutical ingredients, and/or excipients), and based on the comparison, a concentration of the substances in a liquid sample may be accurately determined in real time. In some embodiments, determining the one or more concentrations further comprises detecting one or more substance impurities. Accordingly, non-destructive testing of final dosage forms may be determined and used to provide real-time feedback to an infusion and/or drug delivery system.

As disclosed herewith, a spectroscopy technique is provided by various embodiments of the present disclosure that is non-invasive, which minimizes patient discomfort while ensuring optimal therapeutic levels during treatment. The disclosed spectroscopy technique may also enhance measurement precision and sensitivity for drug concentrations, allowing for continuous monitoring and real-time adjustments to dosing.

It is to be understood that the disclosure is not to be limited to the specific embodiments disclosed, and that modifications and other embodiments are intended to be included within the scope of the appended claims. Although specific terms are employed herein, they are used in a generic and descriptive sense only and not for purposes of limitation, unless described otherwise.

## Claims

1. A system comprising:
a spectroscopic liquid flow device coupled to a liquid flow path, wherein the spectroscopic liquid flow device comprises:
a measurement cell configured to receive at least a portion of the liquid flow path;
a laser emitter configured to (i) provide a dual frequency comb source that comprises two optical frequency combs that are configured to emit laser light at evenly spaced intervals across the spectrum of optical frequencies, (ii) generate a spectrum of optical frequencies, and (iii) emit the spectrum of optical frequencies through the portion of the liquid flow path in the measurement cell; and
a photodetector configured to (i) measure one or more absorption and wavelength characteristics of the spectrum of optical frequencies passed through the portion of the liquid flow path and (ii) generate an output signal based on the one or more absorption and wavelength characteristics; and
a computing system configured to determine one or more substance concentrations in the liquid flow path based on the output signal.

2. The system of claim 1, wherein the liquid flow path is configured to transport a substance from a drug delivery source, wherein the drug delivery source comprises at least one of an infusion pump, a syringe pump, an intravenous bag, or a dialysis machine.

3. The system of claim 1, wherein the photodetector is configured to measure absorption and wavelength characteristics of the spectrum of optical frequencies passed through the portion of the liquid flow path.

4. The system of claim 1, wherein the output signal corresponds to at least one of dosage, concentration, composition, volume, or flow rate of the liquid flow path.

5. The system of claim 1, wherein:
(i) the computing system comprises a machine learning algorithm that is configured to determine at least one of active pharmaceutical ingredients or excipients in the liquid flow path based on the output signal, and
(ii) the computing system is configured to determine the one or more substance concentrations by comparing the output signal with one or more reference spectra.

6. A spectroscopic liquid flow device comprising:
an optical coupling system comprising a laser emitter and a measurement cell, wherein:
(i) the laser emitter provides a dual frequency comb source,
(ii) the laser emitter is configured to emit a laser light comprising a spectrum of optical frequencies,
(iii) the optical coupling system is configured to direct the laser light from the laser emitter through the measurement cell, and
(iv) the measurement cell is configured to receive a liquid flow;
a detection system configured to (i) capture transmitted light from the measurement cell and (ii) generate a data signal representative of one or more measured light spectra from the transmitted light;
a spectral processing unit configured to generate an output signal based on the data signal.

7. The spectroscopic liquid flow device of claim 6, wherein the detection system comprises at least one of a photodetector or a camera system.

8. The spectroscopic liquid flow device of claim 6, wherein the spectral processing unit is configured to interpret the output signal and quantify one or more concentrations of one or more components within the liquid flow.

9. The spectroscopic liquid flow device of claim 6 further comprising a calibration and reference system that is configured to control or vary one or more substance concentrations in the liquid flow based on the output signal.

10. The spectroscopic liquid flow device of claim 9, wherein the calibration and reference system (i) comprises one or more samples or one or more reference spectra, and
(ii) is configured to calibrate the output signal based on the one or more samples or the one or more reference spectra.

11. A computer-implemented method comprising:
generating, by one or more processors, a spectrum of optical frequencies with a laser light from a dual frequency comb source, wherein the laser light is provided at evenly spaced intervals across a spectrum;
generating, by the one or more processors, an output signal based on the spectrum of optical frequencies passing through a measurement cell comprising a liquid flow; and
determining, by the one or more processors, one or more substance concentrations in the liquid flow based on the output signal.

12. The computer-implemented method of claim 11, wherein generating the spectrum of optical frequencies comprises.

13. The computer-implemented method of claim 11, wherein generating the output signal comprises measuring the spectrum of optical frequencies using a detection system that is configured to capture intensity of the spectrum of optical frequencies at a plurality of frequencies.

14. The computer-implemented method of claim 11, wherein determining the one or more substance concentrations comprises comparing one or more measured light spectra corresponding to the output signal to one or more reference spectra that correspond to one or more drugs, medications, active pharmaceutical ingredients, or excipients.

15. The computer-implemented method of claim 11, wherein:
(i) determining the one or more substance concentrations comprises analyzing, using one or more signal processing algorithms, the output signal, and
(ii) the one or more signal processing algorithms comprise a Fourier transform or spectral calibration that identify or quantify absorption features
